# EUROPÄISCHE PATENTSCHRIFT

(11) **EP 0 872 476 B1**
(45) Veröffentlichungstag und Bekanntmachung des Hinweises auf die Patenterteilung: **03.07.2002**
(21) Anmeldenummer: 98102479.7
(22) Anmeldetag: 09.11.1995
(51) Int. Cl.: C07C 255/50

(54) **Verfahren zur Herstellung von 2,6-Dichlor-3-fluor-benzonitril und neue Zwischen-produkte**
Herbicidal substance based on 2,6-dichloro-3-fluoro-benzonitrile and new intermediate compounds
Agent herbicidal basé sur 2,6-dichloro-3-fluoro-benzonitrile et nouveaux intermédiaires composés

(30) Priorität: 22.11.1994 DE 4441419
(43) Veröffentlichungstag der Anmeldung: 21.10.1998
(62) Teilanmeldung aus: 95117655.1
(73) Patentinhaber: BAYER AG, 51368 Leverkusen (DE)
(72) Erfinder: Lantzsch, Reinhard, Dr., 42115 Wuppertal (DE); Bussmann, Werner, Dr., League City, Texas 77573 (US); Käsbauer, Josef, Dr., 42929 Wermelskirchen (DE)

(56) Entgegenhaltungen:
- EP-A- 0 599 241
- EP-A- 0 609 179
- EP-A- 0 657 407
- FR-A- 1 232 614
- CHEMICAL ABSTRACTS, Band 115, Nr. 17, Columbus, Ohio, US, Seite 878, Spalte 2, Zusammenfassung Nr. 182866k, SHIGEHARA ET. AL.: "PREPARATION OF 2,6-DICHLORO-3-FLUOROBENZONITRILE."

## Beschreibung

Die Erfindung betrifft ein Verfahren zur Herstellung von 2,6-Dichlor-3-fluor-benzonitril sowie neue Zwischenprodukte.

2,6-Dichlor-3-fluor-benzonitril ist bereits als Zwischenprodukt für die Herstellung von 2,3,6-Trifluor-benzonitril bekannt geworden (vgl. JP-A 03090057 - zitiert in Chem. Abstracts 115:182866). Die Verwendung der genannten Verbindung als Herbizid ist jedoch bisher nicht bekannt geworden. Bezüglich der Herstellung wird in der zitierten Literatur angegeben, dass zunächst 3-Chlor-2,4-difluor-nitrobenzol mit einem Alkalimetallcyanid unter Bildung von 2-Chlor-3-fluor-6-nitro-benzonitril umgesetzt wird, welches dann durch Umsetzung mit Chlor in 2,6-Dichlor-3-fluor-benzonitril umgewandelt wird. Ausbeute und Qualität des so erhaltenen Produktes sind hierbei nicht immer ganz zufriedenstellend.

Die EP-A 0 609 179 beschreibt die Reaktion von Benzaldehyd, das mit Alkyl oder Phenyl substituiert sein kann, mit Hydroxylaminsulfat und eine nachfolgende Dehydratisierung, wobei die Umsetzung in Propionsäure in Gegenwart eines Propionsäuresalzes durch Erhitzen auf eine Außentemperatur von 140 - 165°C während 2 bis 6 Stunden bei Atmosphärendruck unter gleichzeitigem Abdestillieren des sich bildenden Propionsäure/Wasser-Gemisches bis zur Bildung einer Lösung und nachfolgendem abschließenden Abdestillieren des Propionsäure/Wasser-Gemisches bei 100 - 130°C im Vakuum durchgeführt wird und danach das erhaltene Nitril nach allgemein üblichen Methoden isoliert wird.

Die EP-A 0 599 241 beschreibt ein Verfahren zur Herstellung aromatischer Aldehyde, indem man beispielsweise Benzalchlorid, das mit Fluor, Chlor oder Brom substituiert sein kann, mit einer etwa 1 bis etwa 50 %igen wässrigen Lösung eines oder mehrerer Zinksalze bei Temperaturen von etwa 70 bis etwa 160°C hydrolysiert.

Die FR-A 1 232 614 beschreibt ein spezielles Verfahren zur Herstellung von polychlorierten Benzoesäure-Verbindungen, wobei in einem Teilschritt 2,6-Dichlortoluol-Derivate mit Chlor zu den entsprechenden Benzalchlorid-Derivaten umgesetzt werden.

Die Verbindung 2,6-Dichlor-3-fluor-benzonitril der nachstehenden Formel (I) 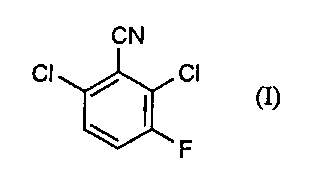 kann sehr gut zur selektiven Bekämpfung von Unkräutern in wichtigen Kulturen verwendet werden.

Es wurde nun gefunden, dass man die Verbindung der Formel (I) erhält, wenn man
a) 2,6-Dichlor-3-fluor-toluol mit Chlor gegebenenfalls in Gegenwart eines Reaktionshilfsmittels bei Temperaturen zwischen 0°C und 100°C umsetzt,
b) das im Schritt a) erhaltene 2,6-Dichlor-3-fluor-benzalchlorid mit Wasser in Gegenwart von Hydrogenchlorid und Eisen(III)-chlorid bei Temperaturen zwischen 0°C und 200°C umsetzt,
c) das im Schritt b) erhaltene 2,6-Dichlor-3-fluor-benzaldehyd mit Hydroxylamin oder einem Säureaddukt gegebenenfalls in Gegenwart eines Verdünnungsmittels und gegebenenfalls in Gegenwart eines Säureakzeptors bei Temperaturen zwischen 0°C und 100°C umsetzt, und
d) das im Schritt c) erhaltene 2,6-Dichlor-3-fluor-benzaloxim der Formel (II) 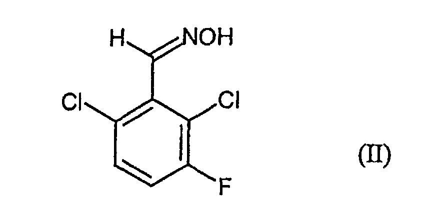 mit einem wasser-entziehenden Mittel bei Temperaturen zwischen 0°C und 300°C im Druckbereich zwischen 0,001 bar und 10 bar umsetzt.

Überraschenderweise kann die hierin beschriebene Verbindung der Formel (I) nach dem erfindungsgemäßen Herstellungsverfahren wesentlich einfacher und auf Basis gut zugänglicher Ausgangsstoffe in sehr guten Ausbeuten hergestellt werden.

Die Verbindung der Formel (II) ist noch nicht aus der Literatur bekannt; sie ist als neuer Stoff Gegenstand der vorliegenden Anmeldung.

Man erhält die neue Verbindung der Formel (II), wenn man 2,6-Dichlor-3-fluorbenzaldehyd der Formel (IV) 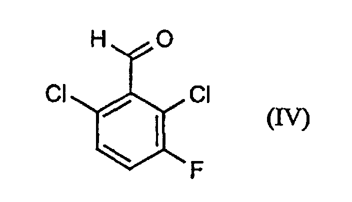 mit Hydroxylamin oder einem Säureaddukt hiervon, wie z.B. Hydroxylamin-Hydrochlorid, gegebenenfalls in Gegenwart eines Verdünnungsmittels, wie z.B. Methanol, Ethanol und/oder Wasser, und gegebenenfalls in Gegenwart eines Säureakzeptors, wie z.B. Natriumacetat, bei Temperaturen zwischen 0°C und 100°C umsetzt (vgl. die Herstellungsbeispiele).

Der als Vorprodukt zu verwendende 2,6-Dichlor-3-fluor-benzaldehyd der Formel (IV) ist noch nicht aus der Literatur bekannt und ist als neuer Stoff Gegenstand der vorliegenden Anmeldung.

Man erhält die neue Verbindung der Formel (IV), wenn man 2,6-Dichlor-3-fluorbenzalchlorid der Formel (VI) 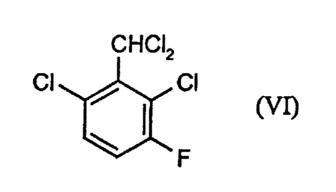 mit Wasser, gegebenenfalls in Gegenwart eines Reaktionshilfsmittels, wie z.B. Hydrogenchlorid und/oder Eisen(III)-chlorid, bei Temperaturen zwischen 0°C und 200°C umsetzt (vgl. die Herstellungsbeispiele).

Das hierbei als Vorprodukt benötigte 2,6-Dichlor-3-fluor-benzalchlorid der Formel (VI) ist noch nicht aus der Literatur bekannt und ist als neuer Stoff ebenfalls Gegenstand der vorliegenden Anmeldung.

Man erhält die neue Verbindung der Formel (VI), wenn man 2,6-Dichlor-3-fluortoluol der Formel (VII) 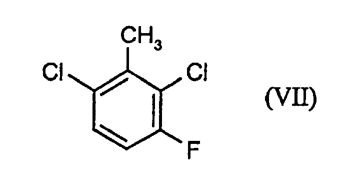 mit Chlor, gegebenenfalls in Gegenwart eines Reaktionshilfsmittels, wie z.B. Pyridin, bei Temperaturen zwischen 0°C und 100°C umsetzt (vgl. die Herstellungsbeispiele).

2,6-Dichlor-3-fluor-toluol der Formel (VII) ist noch nicht aus der Literatur bekannt, ist jedoch als neuer Stoff Gegenstand einer vorgängigen Patentanmeldung.

Man erhält die Verbindung der Formel (VII), wenn man 3-Fluor-toluol der Formel (VIII) 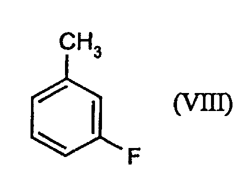 mit Chlor, gegebenenfalls in Gegenwart eines Reaktionshilfsmittels, wie z.B. Eisen-(III)-chlorid und 2,3-Dihydro-5H-benzo[b]-1,4-thiazepin-4-on bei Temperaturen zwischen 0°C und 100°C umsetzt (vgl. die Herstellungsbeispiele).

3-Fluor-toluol ist eine handelsübliche Synthesechemikalie.

Das erfindungsgemäße Verfahren zur Herstellung der Verbindung der Formel (I) wird unter Verwendung eines wasser-entziehenden (dehydratisierenden) Mittels durchgeführt. Es kommen hierbei die üblichen zur Dehydratisierung von Aldoximen geeigneten Mittel in Betracht. Als Beispiele hierfür seien Acetanhydrid, Thionylchlorid, Phosphor(V)-oxid, Dicyclohexylcarbodiimid, Cyanurchlorid, Titan(IV)-chlorid und Benzolsulfonsäurechlorid genannt.

Die Reaktionstemperaturen liegen bei der Durchführung des erfindungsgemäßen Verfahrens zur Herstellung der Verbindung der Formel (I) zwischen 0°C und 300°C, vorzugsweise zwischen 20°C und 250°C, insbesondere zwischen 50°C und 200°C.

Das erfindungsgemäße Verfahren zur Herstellung der Verbindung der Formel (I) wird im Druckbereich zwischen 0,001 bar und 10 bar, vorzugsweise zwischen 0,01 bar und 5 bar, insbesondere zwischen 0,1 bar und 2 bar - durchgeführt.

Bei der Durchführung des erfindungsgemäßen Verfahrens zur Herstellung der Verbindung der Formel (I) setzt man im allgemeinen auf 1 Mol Ausgangsverbindung der Formel (II) wenigstens 1 Mol eines wasser-entziehenden Mittels ein. Das wasserentziehende Mittel kann - vorzugsweise bei Einsatz von Acetanhydrid - auch in hohem Überschuss und damit auch als Verdünnungsmittel verwendet werden. Die Umsetzung und die Aufarbeitung erfolgen nach üblichen Methoden (vgl. die Herstellungsbeispiele).

Die erfindungsgemäße Herstellung des hierin beschriebenen Wirkstoffs geht aus den nachfolgenden Beispielen hervor.

### Herstellungsbeispiele:

### Beispiel 1

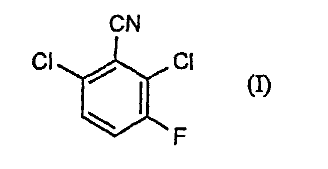

80 g (0,385 Mol) 2,6-Dichlor-3-fluor-benzaldoxim der Formel (II) werden mit 500 ml Acetanhydrid bei Raumtemperatur (ca. 20°C) vermischt. Es bildet sich eine klare Lösung, die dann 6 Stunden lang bei 135°C gerührt wird. Nach Abdestillieren von Essigsäure und Acetanhydrid wird der Rückstand auf Raumtemperatur abgekühlt und in Methylenchlorid aufgenommen. Die Lösung wird dreimal mit Wasser gewaschen, dann mit Natriumsulfat getrocknet und filtriert. Vom Filtrat wird dann das Lösungsmittel unter vermindertem Druck sorgfältig abdestilliert.

Man erhält 67 g (91% der Theorie) 2,6-Dichlor-3-fluor-benzonitril als kristallinen Rückstand vom Schmelzpunkt 69°C-71°C.

### Ausgangsverbindung der Formel (II):

### Beispiel (II-1)

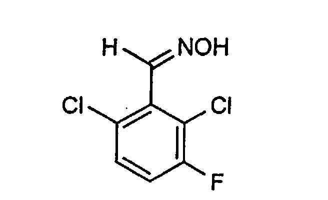

78,3 g (0,383 Mol) 2,6-Dichlor-3-fluor-benzaldehyd (94,5%ig) - in 150 ml Methanol gelöst - werden zu einer Lösung von 31 g (0,446 Mol) Hydroxylamin-hydrochlorid und 36,6 g (0,446 Mol) Natriumacetat in 400 ml Methanol und 40 ml Wasser getropft. Die Reaktion ist exotherm (32°C). Man rührt zwölf Stunden bei Raumtemperatur (20°C) nach und filtriert ab. Der Feststoff wird zweimal mit Wasser ausgerührt und abfiltriert. Schmelzpunkt: 130°C-131°C. Durch Einengen der Mutterlauge und Ausfällen mit Wasser kann weiteres Reaktionsprodukt erhalten werden.

Gesamtausbeute: 80 g (94,8%iges Produkt); Ausbeute: 94% der Theorie.

### Ausgangsverbindung der Formel (IV):

### Beispiel (IV-1)

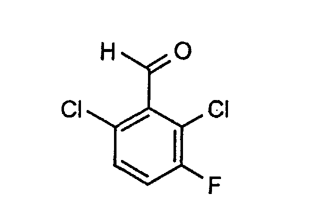

200 g (0,85 Mol) 2,6-Dichlor-3-fluor-benzalchlorid werden in einem Vierhalskolben mit Rührer, Rückflusskühler, Thermometer und Tropftrichter vorgelegt. Dann werden 200 mg Eisen(III)-chlorid - in 23 ml Salzsäure (32%ig) gelöst - innerhalb von 4 Stunden bei 160°C zugetropft. Es wird ein Rohaldehyd mit weniger als 0,7% des Benzalchlorids erhalten.

Nach Destillation im Vakuum (20 mbar) werden 120 g reiner 2,6-Dichlor-3-fluorbenzaldehyd (98,5%ig) erhalten, Ausbeute: 77% der Theorie.

### Ausgangsverbindung der Formel (VI):

### Beispiel (VI-1)

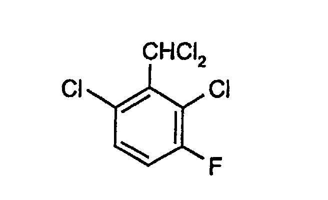

360 g Dichlorfluortoluol-Isomerengemisch (50% 2,6-Dichlor-3-fluor-toluol) werden vorgelegt. Nach Zugabe von 1 Tropfen Pyridin wird Chlor eingeleitet, bis das 2,6-Isomere vollständig in 2,6-Dichlor-3-fluor-benzalchlorid umgewandelt ist. Es liegt dann ein Gemisch mit der ungefähren Zusammensetzung 45% 2,6-Dichlor-3-fluorbenzalchlorid, 45% 4,6-Dichlor-3-fluor-benzalchlorid, 5% 4,6-Dichlor-3-fluor-benzotrichlorid vor.

Dieses wird durch fraktionierte Destillation getrennt. Hierbei wird ein Hauptlauf mit 98,5%igem 2,6-Dichlor-3-fluor-benzalchlorid erhalten, das direkt in die Hydrolysereaktion eingesetzt wird; Ausbeute: 200 g (80% der Theorie).

### Ausgangsverbindung der Formel (VII):

### Beispiel (VII-1)

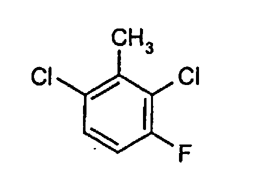

330 g 3-Fluor-toluol werden in einem Vierhalskolben mit Rührer, Rückflusskühler, Thermometer, Gaseinleitungsrohr vorgelegt. Nach Zugabe von 2,3 g Eisen(III)-chlorid und 450 mg 2,3-Dihydro-5H-benzo[b]-1,4-thiazepin-4-on wird bei 20°C unter Rühren Chlor eingeleitet bis nur noch 1 Gewichtsprozent des Monochlorderivates vorhanden ist. Es wird ein Gemisch mit 43% 4,6-Dichlor-3-fluor-toluol und 43 Gewichtsprozent 2,6-Dichlor-3-fluor-toluol erhalten.

Das Reaktionsgemisch wird zweimal mit Wasser (jeweils 60 ml) gewaschen und anschließend destilliert. Dabei wird ein Vorlauf (60 g) erhalten, der im wesentlichen Monochlorfluortoluol enthält und dem nächsten Ansatz zugeführt wird. Als Hauptlauf werden 360 g des Isomeren-Gemisches erhalten, der verbleibende Rückstand wird ebenfalls wieder eingesetzt.

Nach fünffacher Wiederholung liegt die Ausbeute bei 80% der Theorie.

Das erhaltene Isomerengemisch aus 50% 2,6- und 50% 4,6-Dichlor-3-fluor-toluol wird direkt in die nächste Stufe eingesetzt.

## Patentansprüche

1. Verfahren zur Herstellung von 2,6-Dichlor-3-fluor-benzonitril der Formel (I) 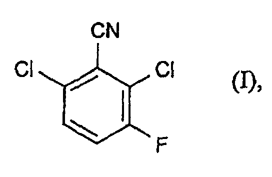 **dadurch gekennzeichnet, daß** man
a) 2,6-Dichlor-3-fluor-toluol mit Chlor gegebenenfalls in Gegenwart eines Reaktionshilfsmittels bei Temperaturen zwischen 0 °C und 100 °C umsetzt,
b) das im Schritt a) erhaltene 2,6-Dichlor-3-fluor-benzalchlorid mit Wasser in Gegenwart von Hydrogenchlorid und Eisen (III)-chlorid bei Temperaturen zwischen 0 °C und 200 °C umsetzt,
c) das im Schritt b) erhaltene 2,6-Dichlor-3-fluor-benzaldehyd mit Hydroxylamin oder einem Säureaddukt gegebenenfalls in Gegenwart eines Verdünnungsmittels und gegebenenfalls in Gegenwart eines Säureakzeptors bei Temperaturen zwischen 0 °C und 100 °C umsetzt, und
d) das im Schritt c) erhaltene 2,6-Dichlor-3-fluor-benzaldoxim der Formel (II) 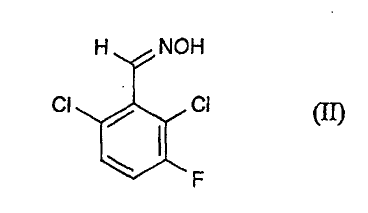 mit einem wasser-entziehenden Mittel bei Temperaturen zwischen 0°C und 300°C im Druckbereich zwischen 0,001 bar und 10 bar umsetzt.

2. 2,6-Dichlor-3-fluor-phenol-Derivate der Formeln

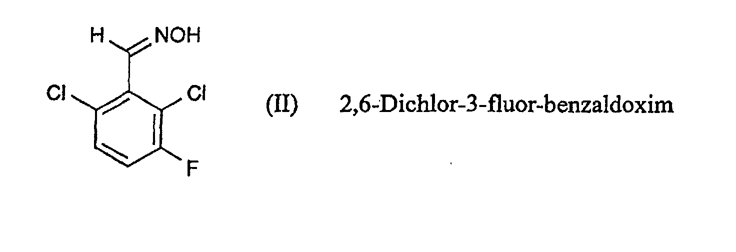

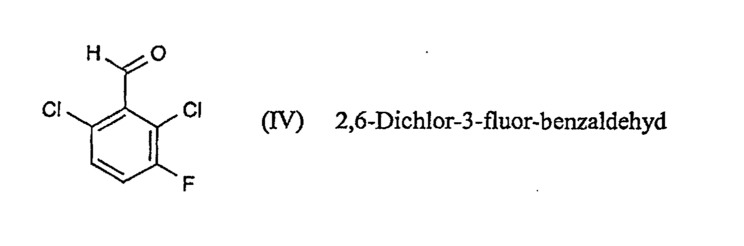

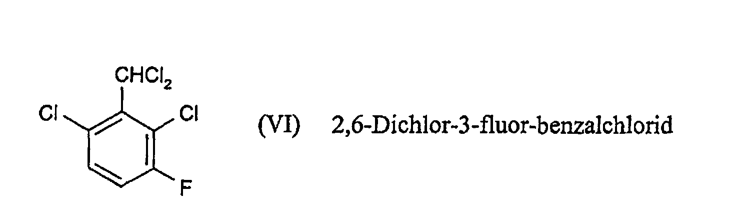

## Claims

1. Process for the preparation of 2,6-dichloro-3-fluoro-benzonitrile of the formula (I) 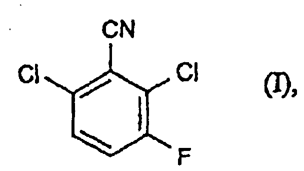 **characterized in that**
a) 2,6-dichloro-3-fluoro-toluene is reacted with chlorine, if appropriate in the presence of a reaction auxiliary, at temperaures of between 0°C and 100°C,
b) the 2,6-dichloro-3-fluoro-benzal chloride obtained in step a) is reacted with water in the presence of hydrogen chloride and iron(III) chloride at temperatures of between 0°C and 200°C,
c) the 2,6-dichloro-3-fluoro-benzaldehyde obtained in step b) is reacted with hydroxylamine or an acid adduct, if appropriate in the presence of a diluent and if appropriate in the presence of an acid acceptor, at temperatures of between 0°C and 100°C, and
d) the 2,6-dichloro-3-fluoro-benzaldoxime obtained in step c) of the formula (II) 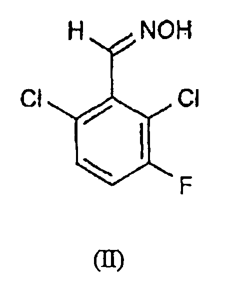 is reacted with a dehydrating agent at temperatures of between 0°C and 300°C in a pressure range of between 0.001 bar and 10 bar.

2. 2,6-Dichloro-3-fluoro-phenol derivatives of the formulae

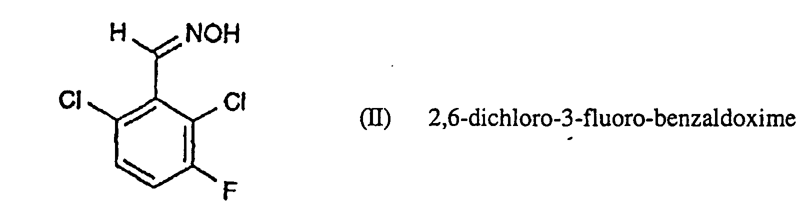

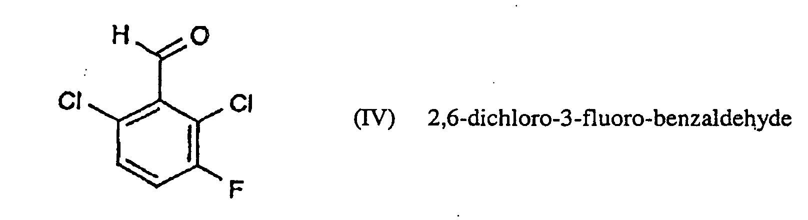

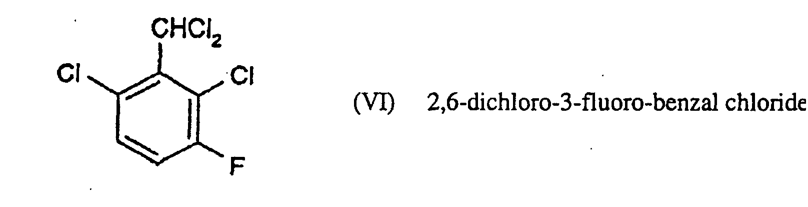

## Revendications

1. Procédé de production de 2,6-dichloro-3-fluoro-benzonitrile de formule (I) 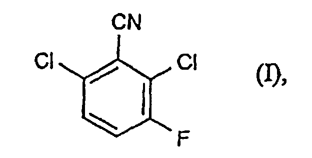 **caractérisé en ce que**
a) on fait réagir le 2,6-dichloro-3-fluoro-toluène avec le chlore, éventuellement en présence d'un auxiliaire de réaction, à des températures comprises entre 0°C et 100°C,
b) on fait réagir le chlorure de 2,6-dichloro-3-fluoro-benzylidène obtenu dans l'étape a) avec l'eau en présence de chlorure d'hydrogène et de chlorure de fer(III) à des températures comprises entre 0°C et 200°C,
c) on fait réagir le 2,6-dichloro-3-fluoro-benzaldéhyde obtenu dans l'étape b) avec l'hydroxylamine ou un produit d'addition d'acide, le cas échéant en présence d'un diluant et en présence éventuelle d'un accepteur d'acide, à des températures comprises entre 0°C et 100°C, et
d) on fait réagir l'oxime de 2,6-dichloro-3-fluoro-benzylidène obtenue dans l'étape c), de formule (II) 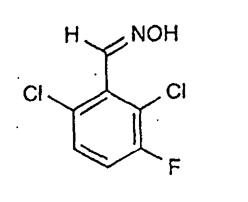 avec un agent déshydratant à des températures comprises entre 0°C et 300°C et dans la plage de pression de 0,001 bar à 10 bars.

2. Dérivés de 2,6-dichloro-3-fluorophénol de formules
